# EUROPEAN PATENT SPECIFICATION

(11) **EP 4 045 220 B1**
(45) Date of publication and mention of the grant of the patent: **03.01.2024**
(21) Application number: 21770288.5
(22) Date of filing: 02.09.2021
(51) Int. Cl.: B23K 9/32, A61F 9/06

(54) **AUTO-DARKENING LCD FILTER GLASS ELEMENT**
AUTOMATISCH VERDUNKELNDES LCD-FILTERGLASELEMENT
ÉLÉMENT EN VERRE À FILTRE LCD AUTO-OBSCURCISSANT

(30) Priority: 19.10.2020 FI 20206025
(43) Date of publication of application: 24.08.2022
(73) Proprietor: Kemppi Oy, 15810 Lahti (FI)
(72) Inventor: MÄKIMAA, Toivo, 15800 Lahti (FI)
(74) Representative: Boco IP Oy Ab
(86) International application number: PCT/FI2021/050591
(87) International publication number: WO 2022/084577

(56) References cited:
- WO-A1-93/13397
- US-A- 4 863 244

## Description

### Field

The present invention relates to an apparatus and a method related to auto-darkening liquid crystal display (LCD) type filter glass elements. More particularly, the invention relates to an apparatus and a method for controlling an automatically darkening LCD filter glass element used in protective welding helmets and welding masks suitable in particular for protecting users during tungsten inert gas (TIG) welding, also known as gas tungsten arc welding (GTAW), and/or during plasma arc welding (PAW) and/or during plasma cutting, see e.g. WO9313397 A1.

### Background

Tungsten Inert Gas (TIG) welding, also known as Gas Tungsten Arc Welding (GTAW) is an arc welding process that produces the weld with a non-consumable tungsten electrode.

The arc created during TIG welding gives off not only visible light, but also UV (ultra-violet) and IR (infra-red) radiation that are absorbed by the cornea and can even reach the retina of the eye. The UV given off by any electric arc welding is considerably more intense than sunlight. Therefore, automatically darkening LCD filter glass elements, referred in the art as auto-darkening LCD elements, are used in welding helmets and welding masks for protecting eyes of the user during operation. Once the weld arc is struck, the auto-darkening LCD element will automatically darken to protect eyes of the user from optical radiation by the weld arc.

Plasma arc welding (PAW) is another known arc welding process, in which an electric arc is formed between a non-consumable electrode and the workpiece. In addition to welding, a plasma arc may also be used for cutting electrically conductive materials by means of an accelerated jet of hot plasma.

Darkening of an LCD filter glass is based on embedding a glass panel with Liquid Crystal Cells (LCC). There may be more than one layer of LCC's, which may further be complemented with polarizing filters. When a weld arc is struck, photo sensors are used to detect presence of bright incident light, and the LCCs in the LCD element are activated with electricity to reduce amount of light passing through the LCD filter glass, thus darkening the panel. When no welding is performed, the LCCs are not activated so that they are in a position that does not reduce amount of light passing through significantly, so that a light lens allows clear visibility and evaluation of the weld piece and the surrounding area.

In practical situations, a problem has been identified that sometimes a TIG arc is not capable of activating the auto-darkening LCD element, thus leaving eyes of the user subject to the harmful optical radiation. In comparison to other common types of welding arcs, intensity of a TIG arc and thus also light emitted by it is rather steady by nature, and in situations in which intensity of the TIG arc and light emitted by it is particularly steady, it can be referred to as a silent TIG arc.

A steady plasma arc used in PAW or in plasma cutting may have like silent arc characteristics as a silent TIG arc. Thus, eyes of a user of PAW or plasma cutting device may also be protected by an auto-darkening LCD element of a welding mask or welding helmet. A PAW arc and a plasma arc used for plasma cutting is hereby commonly referred to as a plasma arc. In the following, the term "silent arc" refers to both a silent TIG arc, a silent plasma arc.

In other known other arc welding methods, such as rod, MIG- or MAG-welding, the welding process itself, in particular transfer of material during the process, causes great changes in both current and voltage used for operating the weld, which are reflected to the light emitted by the weld arc, which demonstrates high variation of intensity. In TIG welding, the welding process itself does not cause such changes caused by transfer of material, and thus the TIG weld arc is more stable than the arc used in rod, MIG and MAG-welding. Several aspects of the welding process and equipment have effect on the stability of the arc, but it is known that TIG welding of some materials, such as high-grade steels or titanium, causes a particularly steady TIG arc.

In addition to the welding process itself, stability of the light emission of the TIG arc is affected by stability of electrical current. Currently, a so-called inverter technology, in which AC power is first rectified to DC, which is then switched (inverted) into a stepdown transformer to produce the desired welding voltage and/or current supplied to generate the arc. In best cases, undulation of load current of an inverter power source is quite small, and the achieved load current is thus stable. When a stable load current is used for generating a TIG arc, the weld arc is also very stable and variation of light emission of the arc is small. Such very steady TIG weld arc may be referred to as a silent TIG arc.

Main reason for the problem is that a TIG arc and/or a plasma arc and in particular a silent TIG arc and/or a silent plasma arc, is not always properly distinguished by the control circuitry of the auto-darkening LCD element from normal ambient light, and thus it does not activate of the auto-darkening LCD element. Increasing sensitivity of the activation of the auto-darkening LCD element by using a lower limit value set for the brightness tends to cause occasional activation of the darkening mode even due to bright normal ambient light, which creates problems in practical use situations.

Thus, an apparatus and a method for recognizing presence of a TIG and/or a plasma arc is needed in all situations, that ensures distinction between ambient light and light emitted by the weld arc for all types of welding, in particular for TIG welding and PAW and also for plasma cutting.

### Description of the related art

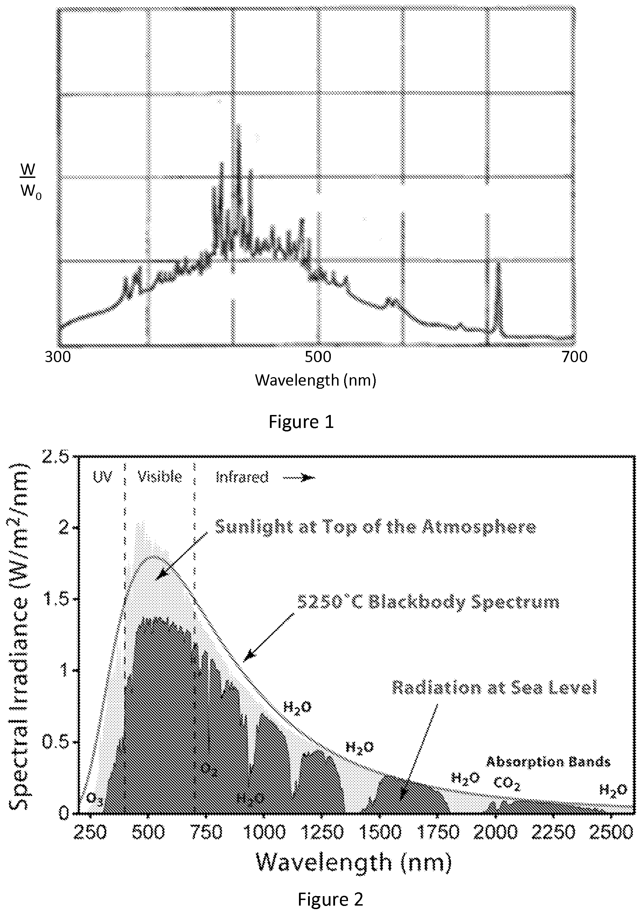

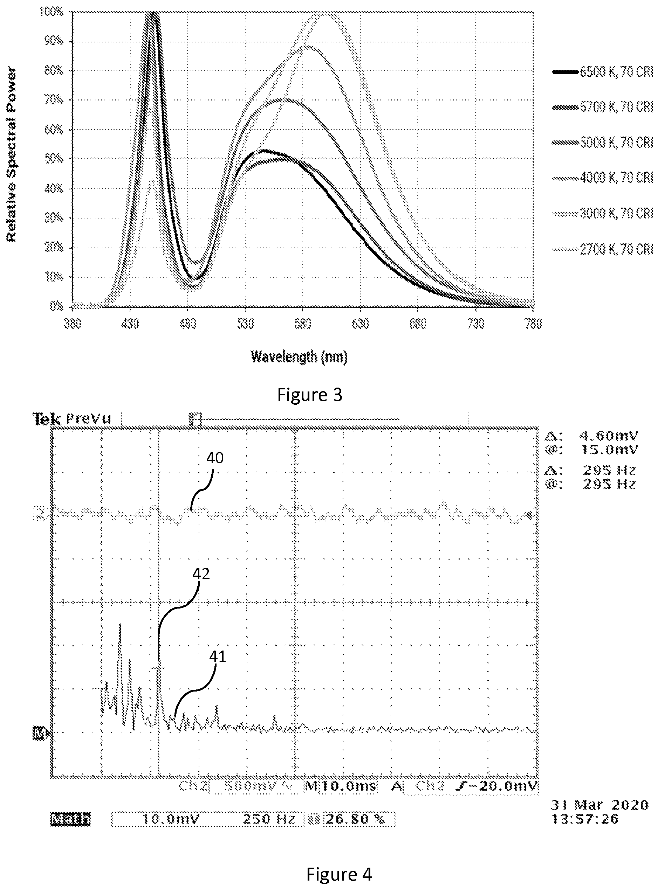

The figure 1 shows an exemplary plot of optical radiation spectrum of light emitted by a TIG arc. Significant components appear on wavelengths between 300 nm and 500 nm, which falls within ultraviolet (UV) range and visible light. Optical radiation spectrum of normal sunlight at sea level has clearly broader bandwidth, extending all way from ultraviolet (UV) to infrared (IR) range, as shown in the figure 2. Intensity maximum of sunlight is at higher wavelengths than that of the TIG arc emitted light. Optical radiation spectrum of an incandescent lamp is rather similar to that of the Sun, with the difference that the peak of the optical radiation spectrum is at about 1000 nm wavelength. Spectral power of typical power LEDs used for lighting is illustrated in the figure 3, with a typical peak power values at about 450 nm range and other range of high-power values in wavelengths above 500 nm.

Thus, it is possible to distinguish light of a TIG arc by detecting ultraviolet light at about 350 nm wavelength, in which the TIG arc spectral characteristics differ from spectral powers of all typical ambient light sources. However, suitable and sufficiently reliable, selective light sensors tend to be difficult to find and quite expensive.

Patent application US20060285330 discloses an automatic darkening filter.

Patent application US20100265421 discloses an automatic darkening filter apparatus comprising a shutter control system switching between different states in response to a change in intensity of incident light.

Patent application US20040178326 discloses a microprocessor based automatically dimmable eye protection with interruption prevention.

### Summary

An object is to provide an apparatus and a method for automatically activating an auto-darkening LCD element. Preferably, this object should be achieved in energy efficient way. In particular the object is to provide an apparatus and a method for automatically activating the auto-darkening LCD element in case of presence of a TIG arc. The objects of the present invention are achieved with an apparatus according to claim 1 and a welding helmet or welding mask according to claim 9. The objects of the present invention are further achieved with a method according to the claim 10.

The preferred embodiments of the invention are disclosed in the dependent claims.

According to a first aspect, an apparatus comprising an auto-darkening LCD element and circuitry for controlling the auto-darkening LCD element is provided. The circuitry comprises a light detector configured to detect intensity of incident light as a function of time and to generate a detection signal on basis of the detected intensity of incident light. The circuitry also comprises a signal processing circuitry configured to process the detection signal for generating a silent arc indication signal indicating presence of a silent arc when amplitude of the detection signal in a predetermined frequency range characteristic to a silent arc is above a predetermined threshold value, and wherein a lower limit of the predetermined frequency range is between 300 Hz and 400 Hz.

The circuitry further comprises an activation circuitry configured to activate darkening of the auto-darkening LCD element when presence of the silent arc is indicated by the silent arc indication signal.

According to a second aspect, said signal processing circuitry comprises a high pass filter configured to filter the detection signal, wherein a cut-off frequency of the high pass filter is at a lower limit of said predetermined frequency range that is characteristic to a silent arc, and wherein the cut-off frequency of the high pass filter is higher than a respective frequency range of intensity variation of light emitted by typical ambient light sources, such as incandescent lamps, fluorescent lamps and/or LED lamps, and a comparator configured to compare amplitude of the filtered detection signal to the predetermined threshold value for generating the silent arc indication signal.

According to a third aspect, the signal processing circuitry further comprises a low pass filter configured to low pass filter the detection signal, the low pass filter having a cut-off frequency between about 1 kHz and about 3 kHz, or a band pass filter configured to perform both said high pass filtering and said low pass filtering.

According to a fourth aspect, the activation circuitry is further configured to activate darkening of the auto-darkening LCD element upon determining, by the circuitry for controlling the auto-darkening LCD element, presence of incident light with an intensity that exceeds a predetermined intensity threshold.

According to a fifth aspect, the circuitry is configured to have a) an active state, in which the circuitry is active, and the auto-darkening LCD element is darkened, b) a standby state, in which the circuitry is active and capable of activating said darkening of the auto-darkening LCD element within about 0.1 to 1 ms from receiving the indication of presence of the silent arc and upon determining presence of incident light with an intensity that exceeds the predetermined intensity threshold, and c) an idle state, in which at least part of the circuitry is set to a low-power state, in which it consumes less power than in the active state.

According to a sixth aspect, the circuitry further comprises a motion detector, wherein the circuitry is configured to be put from the idle state into the standby state upon detecting, by the motion detector, that amount of movement of the apparatus within a predetermined time period exceeds a predetermined movement threshold value, and wherein the circuitry is configured to be put from the standby state into the idle state upon detecting, by the motion sensor, that amount of movement of the apparatus within the predetermined time period does not exceed the predetermined movement threshold value.

According to an seventh aspect, the circuitry further comprises a voltage multiplier configured to generate a trigger voltage for triggering activation of the auto-darkening LCD element, wherein a) in the standby state of the circuitry, the voltage multiplier is configured to operate periodically for generating the trigger voltage, and the circuitry further comprises at least one capacitor configured to store the trigger voltage temporarily, when the voltage multiplier is off during the periodical trigger voltage generation operation, and b) in the idle state of the circuitry, operation of the voltage multiplier is disabled.

According to a eighth aspect, the circuitry for controlling is implemented using CMOS logic.

According to another aspect, a welding helmet or a welding mask is provided, comprising an auto-darkening LCD element and the circuitry for controlling the auto-darkening LCD element according to any one of the preceding claims. According to a first method aspect, a method for controlling an auto-darkening LCD element is provided, the method comprising: i) detecting intensity of incident light as a function of time and generating a detection signal on basis of the detected intensity of incident light; ii) processing said detection signal for generating a silent arc indication signal indicating presence of a silent arc when amplitude of the detection signal in a predetermined frequency range characteristic to a silent arc is above a predetermined threshold value, and wherein a lower limit of the predetermined frequency range is between 300 Hz and 400 Hz, and iii) activating darkening of the auto-darkening LCD element when presence of the silent arc is indicated by the silent arc indication signal.

According to a second method aspect, processing said detection signal comprises high pass filtering the detection signal for extracting a portion of the detection signal that is in a predetermined frequency range that is characteristic to a silent arc, and wherein the predetermined frequency range is above a respective frequency range of intensity variation of light emitted by typical ambient light sources, such as incandescent lamps, fluorescent lamps and/or LED lamps, and comparing amplitude of the filtered detection signal to the predetermined threshold value for generating the silent arc indication signal.

According to a third method aspect, the method further comprises low pass filtering the detection signal, wherein the low pass filtering defines the upper limit of the predetermined frequency range, the upper limit being between about 1 kHz and about 3 kHz.

According to a fourth method aspect, the method comprises activating said darkening of the auto-darkening LCD element also upon determining presence of incident light with an intensity that exceeds a predetermined intensity threshold.

According to a fifth method aspect, the method further comprises setting a circuitry implementing the method into one of: a) an active state, in which the circuitry is active, and the auto-darkening LCD element is darkened, b) a standby state, in which the circuitry is active and capable of activating said darkening of the auto-darkening LCD element within about 0.1 to 1 ms from receiving the indication of presence of the silent arc and upon determining presence of incident light with an intensity that exceeds the predetermined intensity threshold, and c) an idle state, in which at least part of the circuitry is set to a low-power state, in which it consumes less power than in the active state.

According to a sixth method aspect, the circuitry is put from the idle state into the standby state upon determining that amount of movement within a predetermined time period exceeds a predetermined movement threshold value, and the circuitry is put from the standby state into the idle state upon detecting that amount of movement of the apparatus within the predetermined time period does not exceed the predetermined movement threshold value.

According to an seventh method aspect, the method further comprises generating a trigger voltage for triggering activation of the auto-darkening LCD element, wherein a) in the standby state, the trigger voltage is generated periodically, and the trigger voltage is stored temporarily in at least one capacitor during off periods of the periodical trigger voltage generation operation, and b) in the idle state of the circuitry, no trigger voltage is generated.

The present invention is based on the idea of utilizing light intensity variation characteristics of a silent TIG arc and/or a silent plasma arc for detecting and indicating presence of the silent TIG arc and/or the silent plasma arc, and using this information for activating darkening of the auto-darkening LCD element. This operation can complement traditional activation of the auto-darkening LCD element based on detecting presence of bright incident light and activating the darkening when the detected brightness exceeds a brightness threshold value. The present invention has the advantage that it improves reliability of activation of the auto-darkening LCD element and thus improves protection of user's eyes during TIG welding, as well as during PAW and plasma cutting. The invention further improves energy efficiency of circuitry used for controlling the auto-darkening LCD element, which consequently increases battery lifetime and thus operation lifetime of the welding helmet or welding mask.

### Brief description of the drawings

In the following the invention will be described in greater detail, in connection with preferred embodiments, with reference to the attached drawings, in which
Figure 1 illustrates optical radiation spectrum of light emitted by a TIG arc.
Figure 2 illustrates optical radiation spectrum of normal sunlight.
Figure 3 illustrates optical radiation spectrum of power LEDs.
Figure 4 illustrates characteristics of intensity of light emitted by a TIG arc
Figure 5 illustrates characteristics of intensity of light emitted by a LED light source.
Figure 6 illustrates a circuitry for detecting presence of a TIG arc according to the invention.
Figure 7 illustrates an implementation example of a circuitry for detecting and filtering.
Figure 8 illustrates another implementation example of the circuitry for detecting and filtering.
Figure 9 illustrates intensity variation of light emitted by various light sources.
Figure 10 illustrates a detection signal representing intensity of incident light emitted by a TIG silent arc before and after filtering.

### Detailed description

In the following, the invention is described in connection to TIG welding. However, the same finding applies to plasma arc in selected conditions, and the solution is thus equally applicable to PAW and plasma cutting.

Figure 4 illustrates characteristics of intensity of light emitted by a silent TIG arc. Upper graph (40) illustrates intensity in time domain, in other words as function of time, whereas the lower graph (41) illustrates variation of the intensity in frequency domain. Vertical line (42) is in this plot placed at 295 Hz on the lower graph scale, for showing that there is significant variation of the intensity of the silent TIG arc emitted light also at frequencies above 300 Hz.

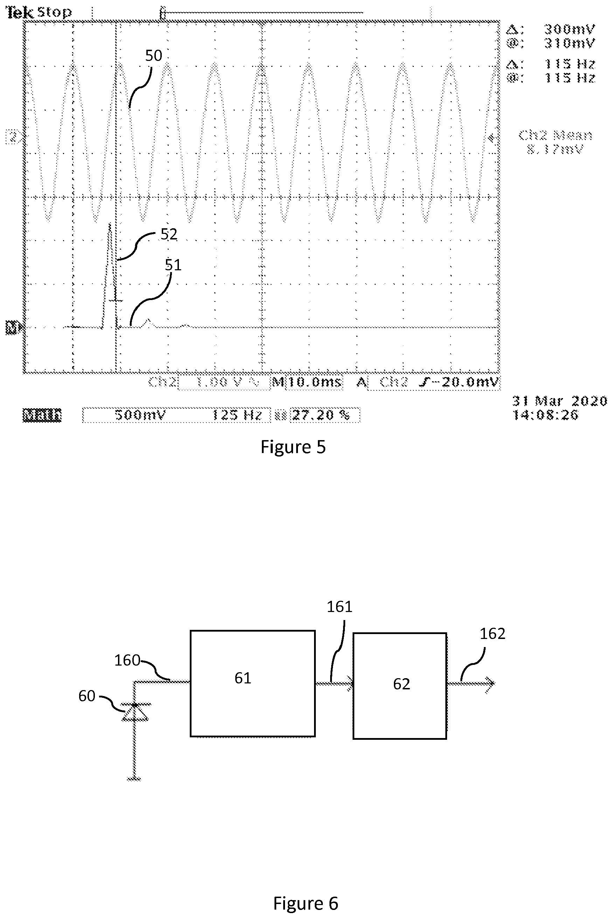

Figure 5 illustrates characteristics of intensity of light emitted by a LED light source. In the time domain, illustrated with the upper graph (50), a clear sine wave-type variation of the intensity can be detected. This is also clearly visible in variation of the intensity of light emitted by the LED light in the frequency domain, as showed in the lower graph (51) having a dominating peak at about 100 Hz, a noticeable overtone at 200 Hz, and another weak overtone at 300Hz. The vertical line marker (52) in this plot is at 115 Hz frequency of the lower graph scale. Variation of intensity of light emitted by incandescent lamp demonstrates even clearer frequency dependency, with a single peak at 100 Hz and no significant intensity variation on higher frequencies. Variation of intensity of light emitted by a fluorescent lamp typically has a dominant peak at 100 Hz, but also a noticeable overtone at 200 Hz.

Sunlight is known to be stable, with no significant variation in intensity over short time periods. Thus, there are no distinctive frequency components in sunlight intensity.

Based on the above findings, the inventors have found that it is possible to distinct light from a silent TIG arc from ambient light by detecting frequency components of variation of intensity of the detected incident light that occurs above about 300 Hz. The same principle is also applicable to a plasma arc used in PAW and plasma cutting. Ideal solution would be a high-pass filter with sharp cut-off frequency, attenuating any frequencies at or below 300 Hz, and little attenuation from 301 Hz onwards. In tests it has been found that a high-pass filter with 50 to 60 dB attenuation at 100 Hz and with no significant attenuation at 400 Hz or higher is capable of providing enough distinction between intensity variations of incident light by ambient light sources and by the silent arc. In practice the lower limit of the useful frequency range falls between 300 Hz and 400 Hz, and also the cut-off frequency of the high pass filter falls on this range. Theoretically, there is no fixed upper limit of the useful frequency range for the silent arc detection purpose, but in practice there are some disturbances with higher frequencies in kHz range, which are preferably filtered out to avoid false positives and thus to improve reliability of detection. Desire for low power consumption also tends to limit the upper limit of the frequency range of active high-pass filters.

While initial testing and design of the exemplary embodiment has been made in Europe, with grid frequency of 50 Hz, it should be noticed, that in countries with gird frequency of 60 Hz, the characteristic intensity variation frequencies of typical ambient light sources are also different, since these are typically integer multiples of the grid frequency. Thus, for use in a country with 60 Hz grid frequency, typical ambient light sources may have characteristic light intensity variation for example on frequencies of 120 Hz, 240 Hz and 360 Hz, in which case the high-pass filter preferably has a cut-off frequency above about 360 Hz.

One implementation alternative is to replace the high-pass filter with specific band-stop filters at typical frequencies of variation of light intensities of the ambient light sources, i.e. at 100 Hz, 200 Hz and optionally 300 Hz in countries with 50 Hz grid frequency or at 120 Hz, 240 Hz and optionally 360 Hz in countries with 60 Hz grid frequency. However, implementing such band pass filters is much more complicated than a simple active high-pass filter, and likely more power consuming.

Figure 6 illustrates an exemplary implementation for a circuitry for detecting presence of a silent arc. The circuitry includes a light detector (60) that provides a detection signal (160) that corresponds to detected intensity of incident light, and a signal processing circuitry for processing the detection signal (160) for producing a TIG arc indication signal (162) that indicates presence of a TIG arc.

Typically, the detection signal is an electrical signal, but also other types of detection signal may be applicable. When the welding helmet or the welding mask with the auto-darkening LCD element and the circuitry for controlling thereof is in use, the light detector (60) preferably operates continuously so that the detection signal provides information on intensity of incident light as a function of time.

The signal processing circuitry may comprise a high pass filter (61) for filtering out any detection signals with frequencies in ranges typical to ambient light sources, and a comparator (62) for providing a silent arc indication signal (162) indicating whether a silent arc is detected or not. As known in the art output of the comparator (62) may be considered as a digital signal.

Cut-off frequency of the high pass filter (61) may be selected to fall at the selected lower limit of the above-mentioned useful frequency range that is particularly characteristic for variation of intensity of light emitted by a silent arc. This is achieved by defining the cut-off frequency of the high pass filter (61) such that it effectively filters frequency components from the detection signal that are typical for ambient light sources, such as LED or incandescent lamp, but passes frequencies that are characteristics to the silent arc. In the preferred, non-limiting embodiment, the cut-off frequency is about 300 Hz, but any cut-off frequency within the above-mentioned frequency ranges of the useful frequency range may be selected. The high pass filter (61) produces a high pass filtered detection signal, which may be further subjected to a low pass filter (not shown) for removing other unwanted frequency components. The resulting filtered detection signal (161) comprises frequencies which are characteristic to a variation of intensity of light emitted by a silent arc, but not typical for any common ambient light sources. Instead of separate high pass and low pass filters, the detection signal (160) may be filtered using a bandpass filter with a pass band suitable for this purpose to produce the filtered detection signal (161). Depending on implementation, the high pass filtered detection signal as such may be used as the detection signal (161).

The filtered detection signal (161) is then fed to the comparator (62), which compares amplitude of the voltage of the filtered detection signal to a predetermined threshold value. If the amplitude of the filtered detection signal exceeds the predetermined threshold value, the silent arc indication signal (162) at the output of the comparator (62) indicates that a silent arc is detected. If the amplitude is below the predetermined threshold value, it is deemed that no silent arc was detected. As known in the art, a comparator outputs a digital type signal with two alternative values. This silent arc indication signal (162) can subsequently be used to control the activation circuitry that activates the auto-darkening LCD element, which can also be referred to as darkening of the auto-darkening LCD element.

Control circuitry that activates darkening of the auto-darkening LCD element preferably implements hysteresis for avoiding blinking of the auto-darkening LCD element between its "light" and "darkened" states. Hysteresis may be implemented for example by introducing a delay for deactivating the auto-darkening LCD element. Thus, a short break in the silent arc or any other welding or plasma cutting arc does not immediately deactivate the auto-darkening LCD element. In an exemplary implementation, an about 1 second delay may be applied before it is determined that no more silent arc and/or any other type of welding arc is detected, after which deactivation occurs.

For other types of weld arcs, it is typically sufficient to detect brightness of incident light and use presence of bright incident light as the main physical quantity on which activating the auto-darkening LCD element is based. For implementing a generic use auto-darkening LCD element suitable to be used with various types of welding equipment, the circuitry is preferably further configured to detect presence of bright incident light produced by any other type of weld arc and detected by the light detector or a further light detector. This kind of circuitries used for detecting presence of a welding arc based on detection of bright incident light are well known in the art and may easily be combined with the invented circuitry for controlling a multi-purpose auto-darkening LCD element.

In a practical implementation, signal of the light detector (60) will likely need to be amplified in addition to filtering. Any suitable amplifier circuitry known in the art may be used.

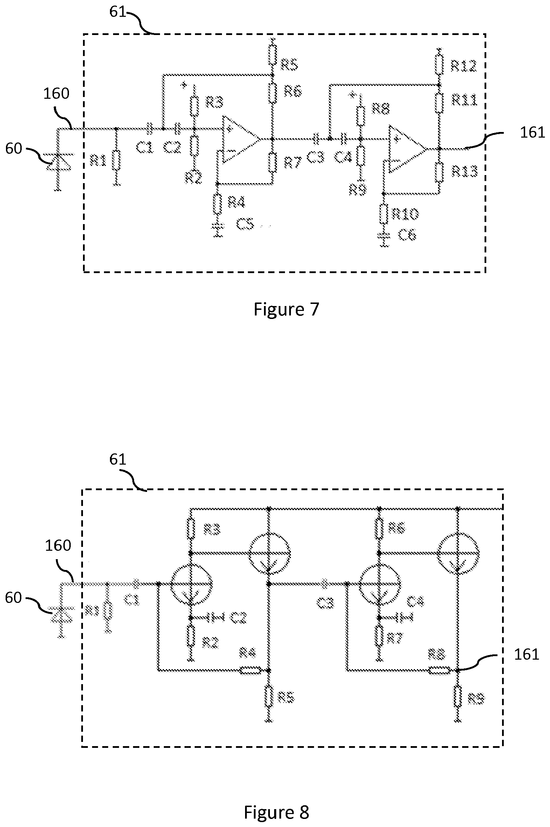

Figure 7 illustrates one possible, non-limiting implementation example of the circuitry for detecting intensity of incident light and for filtering the detection signal which may be utilized in detecting presence of a silent arc, in particular a silent TIC arc. The light detector (60) is implemented with a photodiode for providing the detection signal (160) and the high pass filter (61) is implemented using an active two-stage filter circuitry for providing the filtered detection signal (161). Output of the high pass filter may further be fed through a low-pass filter (not shown) before using a comparator (not shown) for determining whether a silent arc is detected as explained above. Output of the comparator may be used as indicator signal for indicating the presence of the silent arc to circuitry controlling activation of the auto-darkening LCD element.

Figure 8 illustrates another non-limiting implementation example of the circuitry for detecting intensity of incident light and for filtering the detection signal which may be utilized in detecting presence of a silent arc. In comparison to the circuitry of figure 7, this high-pass filter (61) circuitry has been designed for power saving.

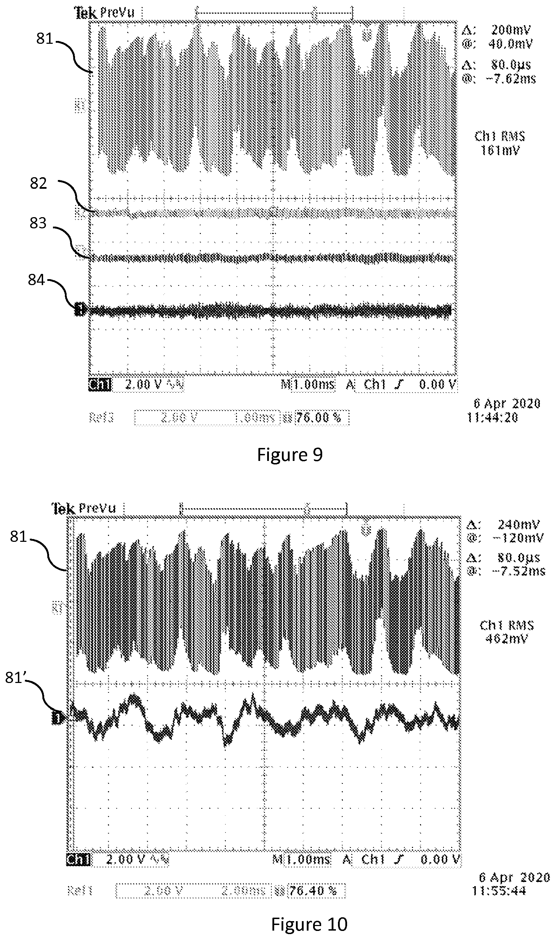

Figure 9 illustrates filtered detection signals (161) at the output of the exemplary high pass filter (61) with different types of light emission sources detected by the light detector (60). Graph 81 represents a high pass filtered detection signal at the output of the high pass filter (61) obtained on basis of a 10A DC TIG arc operated with an inverter power source. Graph 82 represents a correspondingly filtered detection signal obtained on basis of light emitted by a fluorescent lamp, graph 83 represents a correspondingly filtered detection signal obtained on basis of light emitted by an incandescent lamp and graph (84) represents a correspondingly filtered detection signal obtained on basis of light emitted by a LED lighting device.

The graph 81 indicates that the detection signal (81) representing intensity of incident light emitted by the silent arc includes at least one higher frequency component. In the exemplary system, this higher frequency component is caused by an inverter that is used for generating a DC power signal for operating the TIG weld. If such component is produced in the used welding apparatus, the higher frequency component can be further filtered out for example by using a low pass filter that filters out the inverter frequency. Exact inverter frequency to be filtered out is a design option, depending on the source and characteristics of such higher frequency component, such as implementation of inverter apparatus used. In the exemplary system, a low pass filter with a cut-off frequency at about 3 kHz was used.

Figure 10 illustrates in time domain the detection signal representing intensity of incident light emitted by a silent arc before (81) and after (81') further low pass filtering. Low pass filtering effectively removes the distorting high frequency component from the detection signal (81) in which presence of frequency components above 300 Hz are clearly visible, and the remaining filtered detection signal (81') is ready to be used for determining whether a silent arc was detected for example using the comparator as explained above. Depending on the implementation, low pass filter cut-off frequency may be for example between 1 kHz and 3 kHz, preferably between 2 kHz and 3 kHz. In some embodiments, the high pass filter and the low pass filter may be implemented as a combined band pass filter with suitable upper and lower cut-off frequencies.

Although the above description uses TIG welding and in particular a silent TIG arc as an example, the same principle applies to a plasma arc used in PAW or in plasma cutting.

When designing a practical product, and energy efficiency of the implementation of the auto-darkening LCD element and the circuitry controlling its operation is another aspect to consider. An active photodiode, detection circuitry and filtering circuitry all consume energy. Although the welding helmet or the welding mask with auto-darkening LCD element requires electrical energy for operation, it should preferably be operated using a battery so that no external energy source connection cable is required. An energy efficient implementation facilitates extended battery lifetime.

In a standby state the auto-darkening LCD element is not darkened. Energy is consumed by a light detector and an amplifier associated therewith and a control logic. For facilitating quick darkening operation of the auto-darkening LCD element, an about 20V to 30V voltage peak, referred here as a trigger voltage, is fed to the LCD element. There is a need of darkening the auto-darkening LCD element before human eye can actually detect the light emitted by the arc. Thus, activation of the auto-darkening LCD element shall occur within 0.1 ms to 1 ms from time of appearance of the arc. Thus, the trigger voltage shall be available very quickly. The trigger voltage is in practice obtained from about 3V to 6V battery carried by the welding helmet or the weld mask, and thus it is not readily available. For enabling fast generation of the trigger voltage, energy is consumed during the standby state. In operation, when the auto-darkening LCD element is dark, only about 2V to 3V voltage is required for maintaining the auto-darkening LCD element in the activated state, in which the LCD element is darkened. Energy consumption of the activated, darkened auto-darkening LCD element is higher than in the standby state, since the glass-based LCD element is equivalent to a lossy capacitor, and it requires constant feeding of energy to remain in the active state. Typical current consumption of an auto-darkening LCD element in the active state is about 100 µA-200 µA, or even more. Current consumption of the control logic depends on implementation. A traditional CMOS logic has low standby state current consumption, typically less than 1 µA. If a processor is used for implementing control logic, it may require up to 100 µA-200 µA operating current even in the standby state.

For minimizing the power consumption and maximizing battery lifetime, all power consuming elements of the welding helmet or glasses may be optimized.

For minimizing power consumption of a photodiode used as the light detector, it is beneficial to operate it in photovoltaic mode, also known as the zero-bias mode, so that it does not require external power for operation. In the photovoltaic mode, the photodiode produces a voltage when exposed to light. Photodiode could also be used in photoconductive mode or in avalanche diode mode, both of which uses reverse biasing, but these modes require generation of the reverse bias voltage, which consumes energy.

As explained above, darkening the auto-darkening LCD element quickly enough to protect the user's eyes requires a trigger voltage. Because of the high reaction speed requirement, the trigger voltage needs to be generated in advance so that it is readily available when needed. A typical energy source available is a 3V to 6V battery, and the trigger voltage may be generated based on the battery voltage with a voltage multiplier. Current consumption of a traditional voltage multiplier is in range of hundreds of microamperes. Total power consumption of the voltage multiplier may be reduced for example by operating it periodically. In periodical operation, voltage multiplier only operates periodically. The voltage multiplier operates actively during its active periods, and between the active periods, there are off periods, during which the generated trigger voltage is stored into a capacitor or capacitors from which it is immediately available. Exact periodicity of the periodical trigger voltage generation operation and design and proportioning of the circuitry elements needed are design options.

Energy consumption of the operating logic can be kept low by utilizing CMOS circuitry and low power comparators. If the control operation was so complicated that it requires use of a microprocessor, the microprocessor used should be capable of being put into a low-power idle state whenever the auto-darkening LCD element is not in use, since even low-power microprocessors typically have hundreds of microamperes current consumption in active state. However, future developments in low-power microprocessor technology may provide devices which are able to perform all required signal and data processing steps in power efficient way and thus suitable for implementing functions of the circuitry according to the embodiments.

Because the welding helmet or mask is in practice used only for about 5% of time, a motion sensor may be used for detecting whether the welding helmet or mask is moving, which indicates whether the welding helmet or mask in use or not. If no motion is detected during a predetermined time period, the welding helmet or mask may be put in a deactivated state, which may be also called as an idle state. Any known type of motion sensor may be utilized for detecting movement, for example an acceleration sensor or a gyroscope. In practice, an acceleration sensor of any known type is preferred for implementing motion detection. The gyroscope, although technically equally useful, requires more power and is more expensive. During the idle state, the light detector, some parts of the control logic and/or the trigger voltage generation may be deactivated when the welding helmet or mask is not in use, and activated only if the motion sensor detects that the welding helmet moves sufficiently in a predefined period of time. Thus, only the motion sensor and circuitry monitoring signals from the motion sensor need to be active in the idle state. When movement is detected, the standby state is activated so that the auto-darkening LCD element is ready to be put in active state for protecting eyes of the user. In the standby state, arc detection is active, and the auto-darkening LCD element is activated and darkened upon detecting presence of an arc on basis of characteristics of the incident light as explained above. Vice versa, if after use no more movement is detected during the predefined period of time, the apparatus is put from the standby state to idle state for power saving.

A common battery of a welding helmet is a lithium battery with total capacity of 200 to 600 mAh. Many welding helmets further comprise one or more solar panels as an additional energy source. In normal ambient light, the solar panels typically produce just a little if any energy. During welding action, a well-designed and sufficiently large solar panel may generate several hundreds of microamperes current from the from the incident light energy from the weld arc itself, which may be sufficient to self-produce all energy required for operating the auto-darkening LCD element as well as operation of the circuitry in the welding helmet or mask for controlling and operating it. When the total standby state current consumption is maintained in less than 20 µA and active state current consumption is less than 150 µA, the battery lifetime may be more than one year. With a low power motion sensor, the standby current may be further reduced.

It is apparent to a person skilled in the art that as technology advanced, the basic idea of the invention can be implemented in various ways. The invention and its embodiments are therefore not restricted to the above examples, but they may vary within the scope of the claims.

## Claims

1. An apparatus comprising an auto-darkening LCD element and circuitry for controlling the auto-darkening LCD element, wherein silent arc refers to a situation in which intensity of light emitted by an arc used for TIG welding, plasma arc welding or plasma cutting is particularly steady, wherein the circuitry comprises:
- a light detector (60) configured to detect intensity of incident light as a function of time and to generate a detection signal (160) on basis of the detected intensity of incident light,
**characterized in that** the circuitry comprises:
- a signal processing circuitry (61, 62) configured to process the detection signal (160) for generating a silent arc indication signal (162) indicating presence of a silent arc when variation of amplitude of the detection signal (160) in a predetermined frequency range characteristic to the silent arc is above a predetermined threshold value, and wherein a lower limit of the predetermined frequency range is between 300 Hz and 400 Hz, and
- an activation circuitry configured to activate darkening of the auto-darkening LCD element when presence of the silent arc is indicated by the silent arc indication signal (162).

2. The apparatus according to claim 1, wherein said signal processing circuitry (61, 62) comprises:
- a high pass filter (61) configured to filter the detection signal (160), wherein a cut-off frequency of the high pass filter (61) is at a lower limit of said predetermined frequency range that is characteristic to a silent arc, and wherein the cut-off frequency of the high pass filter (61) is higher than a respective frequency range of intensity variation of light emitted by typical ambient light sources, such as incandescent lamps, fluorescent lamps and/or LED lamps, and
- a comparator (62) configured to compare amplitude of the filtered detection signal (161) to the predetermined threshold value for generating the silent arc indication signal.

3. The apparatus according to claim 1 or 2, wherein the signal processing circuitry further comprises a low pass filter configured to low pass filter the detection signal, the low pass filter having a cut-off frequency between about 1 kHz and about 3 kHz, or a band pass filter configured to perform both said high pass filtering and said low pass filtering.

4. The apparatus according to any of the preceding claims, wherein the activation circuitry is further configured to activate darkening of the auto-darkening LCD element upon determining, by the circuitry for controlling the auto-darkening LCD element, presence of incident light with an intensity that exceeds a predetermined intensity threshold.

5. The apparatus according to any of the preceding claims, wherein the circuitry is configured to have
a) an active state, in which the circuitry is active, and the auto-darkening LCD element is darkened,
b) a standby state, in which the circuitry is active and capable of activating said darkening of the auto-darkening LCD element within about 0.1 to 1 ms from receiving the indication of presence of the silent arc and, when dependent on the claim 4, upon determining presence of incident light with an intensity that exceeds the predetermined intensity threshold, and
c) an idle state, in which at least part of the circuitry is set to a low-power state, in which it consumes less power than in the active state.

6. The apparatus according to claim 5, wherein the circuitry further comprises a motion detector, wherein the circuitry is configured to be put from the idle state into the standby state upon detecting, by the motion detector, that amount of movement of the apparatus within a predetermined time period exceeds a predetermined movement threshold value, and wherein the circuitry is configured to be put from the standby state into the idle state upon detecting, by the motion detector, that amount of movement of the apparatus within the predetermined time period does not exceed the predetermined movement threshold value.

7. The apparatus according to any of claims 5 to 6, wherein the circuitry further comprises a voltage multiplier configured to generate a trigger voltage for triggering activation of the auto-darkening LCD element, wherein
a) in the standby state of the circuitry, the voltage multiplier is configured to operate periodically for generating the trigger voltage, and the circuitry further comprises at least one capacitor configured to store the trigger voltage temporarily, when the voltage multiplier is off during the periodical trigger voltage generation operation, and
b) in the idle state of the circuitry, operation of the voltage multiplier is disabled.

8. A welding helmet or a welding mask comprising an auto-darkening LCD element and the circuitry for controlling the auto-darkening LCD element according to any one of the preceding claims.

9. A method for controlling an auto-darkening LCD element, wherein a silent arc refers to a situation in which intensity of light emitted by an arc used for TIG welding, plasma arc welding or plasma cutting is particularly steady, the method comprising:
- detecting intensity of incident light as a function of time and generating a detection signal (160) on basis of the detected intensity of incident light;
**characterized in that** the method comprises:
- processing said detection signal (160) for generating a silent arc indication signal (162) indicating presence of a silent arc when variation of amplitude of the detection signal (160) in a predetermined frequency range characteristic to a silent arc is above a predetermined threshold value, and wherein a lower limit of the predetermined frequency range is between 300 Hz and 400 Hz, and
- activating darkening of the auto-darkening LCD element when presence of the silent arc is indicated by the silent arc indication signal (162).

10. The method according to claim 9, wherein processing said detection signal (160) comprises:
- high pass filtering the detection signal for extracting a portion of the detection signal that is in a predetermined frequency range that is characteristic to a silent arc, and wherein the predetermined frequency range is above a respective frequency range of intensity variation of light emitted by typical ambient light sources, such as incandescent lamps, fluorescent lamps and/or LED lamps, and
- comparing amplitude of the filtered detection signal (161) to the predetermined threshold value for generating the silent arc indication signal (162).

11. The method according to claim 9 or 10, further comprising low pass filtering the detection signal, wherein the low pass filtering defines the upper limit of the predetermined frequency range, the upper limit being between about 1 kHz and about 3 kHz.

12. The method according to any of claims 9 to 12, further comprising activating said darkening of the auto-darkening LCD element also upon determining presence of incident light with an intensity that exceeds a predetermined intensity threshold.

13. The method according to any of claims 9 to 13, further comprising setting a circuitry implementing the method into one of:
a) an active state, in which the circuitry is active, and the auto-darkening LCD element is darkened,
b) a standby state, in which the circuitry is active and capable of activating said darkening of the auto-darkening LCD element within about 0.1 to 1 ms from receiving the indication of presence of the silent arc and, when dependent on the claim 12, upon determining presence of incident light with an intensity that exceeds the predetermined intensity threshold, and
c) an idle state, in which at least part of the circuitry is set to a low-power state, in which it consumes less power than in the active state.

14. The method according to claim 13, wherein the circuitry is put from the idle state into the standby state upon determining that amount of movement within a predetermined time period exceeds a predetermined movement threshold value, and the circuitry is put from the standby state into the idle state upon detecting that amount of movement of the apparatus within the predetermined time period does not exceed the predetermined movement threshold value.

15. The method according to any of claims 13 to 14, wherein the method further comprises generating a trigger voltage for triggering activation of the auto-darkening LCD element, wherein
a) in the standby state, the trigger voltage is generated periodically, and the trigger voltage is stored temporarily in at least one capacitor during off periods of the periodical trigger voltage generation operation, and
b) in the idle state of the circuitry, no trigger voltage is generated.

## Patentansprüche

1. Vorrichtung umfassend ein selbstverdunkelndes LCD-Element und eine Schaltungsanordnung zur Ansteuerung des selbstverdunkelnden LCD-Elements, wobei ein ruhiger Lichtbogen eine Situation bezeichnet, in der die Intensität von Licht, das von einem beim TIG-Schweißen, Plasmalichtbogenschweißen oder Plasmaschneiden verwendeten Lichtbogen emittiert wird, besonders stet ist, wobei die Schaltungsanordnung umfasst:
- einen Lichtdetektor (60), der dazu ausgelegt ist, die Intensität von einfallendem Licht in Abhängigkeit von der Zeit zu detektieren und ein Detektionssignal (160) basierend auf der detektierten Intensität des einfallenden Lichts zu erzeugen,
**gekennzeichnet dadurch, dass** die Schaltungsanordnung umfasst:
- eine Signalverarbeitungsschaltung (61, 62), die ausgelegt ist zum Verarbeiten des Detektionssignals (160) zur Erzeugung eines Ruhig-Lichtbogen-Anzeigesignals (162), das das Vorliegen eines ruhigen Lichtbogens anzeigt, wenn die Amplitudenschwankung des Detektionssignals (160) in einem für den ruhigen Lichtbogen charakteristischen vorherbestimmten Frequenzbereich einen vorherbestimmten Schwellwert übersteigt, und wobei eine Untergrenze des vorherbestimmten Frequenzbereichs zwischen 300 Hz und 400 Hz beträgt, und
- eine Aktivierungsschaltung, die dazu ausgelegt ist, das Verdunkeln des selbstverdunkelnden LCD-Elements zu aktivieren, wenn vom Ruhig-Lichtbogen-Anzeigesignal (162) das Vorliegen des ruhigen Lichtbogens angezeigt wird.

2. Vorrichtung nach Anspruch 1, wobei die Signalverarbeitungsschaltung (61, 62) umfasst:
- ein Hochpassfilter (61), das zum Filtern des Detektionssignals (160) ausgelegt ist, wobei eine Grenzfrequenz des Hochpassfilters (61) an einer Untergrenze des für einen ruhigen Lichtbogen charakteristischen vorherbestimmten Frequenzbereichs liegt, und wobei die Grenzfrequenz des Hochpassfilters (61) höher ist als ein entsprechender Frequenzbereich der Intensitätsschwankung eines von typischen Umgebungslichtquellen, wie von Glühlampen, fluoreszierenden Lampen und/oder LED-Lampen, emittierten Lichts, und
- einen Vergleicher (62), der dazu ausgelegt ist, die Amplitude des gefilterten Detektionssignals (161) mit dem vorherbestimmten Schwellwert zu vergleichen, um das Ruhig-Lichtbogen-Anzeigesignal zu erzeugen.

3. Vorrichtung nach Anspruch 1 oder 2, wobei die Signalverarbeitungsschaltung ferner umfasst: ein Tiefpassfilter, das zur Tiefpassfilterung des Detektionssignals ausgelegt ist, wobei das Tiefpassfilter eine Grenzfrequenz zwischen etwa 1 kHz und etwa 3 kHz aufweist, oder ein Bandpassfilter, das zur Ausführung sowohl der Hochpassfilterung als auch der Tiefpassfilterung ausgelegt ist.

4. Vorrichtung nach einem der vorhergehenden Ansprüche, wobei die Aktivierungsschaltung ferner dazu ausgelegt ist, das Verdunkeln des selbstverdunkelnden LCD-Elements zu aktivieren, wenn von der Schaltung zur Ansteuerung des selbstverdunkelnden LCD-Elements das Vorliegen von einfallendem Licht mit einer eine vorherbestimmte Intensitätsschwelle überschreitenden Intensität festgestellt wird.

5. Vorrichtung nach einem der vorhergehenden Ansprüche, wobei die Schaltungsanordnung so ausgelegt ist, dass sie Folgendes aufweist:
a) einen Aktiv-Zustand, in dem die Schaltungsanordnung aktiv ist und das selbstverdunkelnde LCD-Element verdunkelt wird,
b) einen Standby-Zustand, in dem die Schaltungsanordnung aktiv und in der Lage ist, die Verdunkelung des selbstverdunkelnden LCD-Elements binnen etwa 0,1 bis 1 ms nach Empfang der Anzeige über das Vorliegen des ruhigen Lichtbogens und, in Abhängigkeit von Anspruch 4, bei Feststellung des Vorliegens von einfallendem Licht mit einer die vorherbestimmte Intensitätsschwelle übersteigenden Intensität, zu aktivieren, und
c) einen Ruhezustand, in dem die Schaltungsanordnung mindestens zum Teil in einen stromsparenden Zustand versetzt wird, in dem sie weniger Strom als im Aktiv-Zustand verbraucht.

6. Vorrichtung nach Anspruch 5, wobei die Schaltungsanordnung ferner einen Bewegungsdetektor umfasst, wobei die Schaltungsanordnung so ausgelegt ist, dass sie vom Ruhezustand in den Standby-Zustand versetzt wird, wenn vom Bewegungsdetektor detektiert wird, dass der Bewegungsbetrag der Vorrichtung innerhalb einer vorherbestimmten Zeitperiode einen vorherbestimmten Bewegungsschwellwert überschreitet, und wobei die Schaltungsanordnung so ausgelegt ist, dass sie vom Standby-Zustand in den Ruhezustand versetzt wird, wenn vom Bewegungsdetector detektiert wird, dass der Bewegungsbetrag der Vorrichtung innerhalb der vorherbestimmten Zeitperiode den vorherbestimmten Bewegungsschwellwert nicht überschreitet.

7. Vorrichtung nach einem der Ansprüche 5 bis 6, wobei die Schaltungsanordnung ferner einen Spannungsvervielfacher umfasst, der dazu ausgelegt ist, eine Triggerspannung zur Auslösung der Aktivierung des selbstverdunkelnden LCD-Elements zu erzeugen, wobei
a) im Standby-Zustand der Schaltungsanordnung der Spannungsvervielfacher für einen periodischen Betrieb ausgelegt ist, um die Triggerspannung zu erzeugen, und die Schaltungsanordnung ferner mindestens einen Kondensator umfasst, der für eine temporäre Speicherung der Triggerspannung ausgelegt ist, wenn der Spannungsvervielfacher während des periodischen Triggerspannungserzeugungsbetriebs ausgeschaltet ist, und
b) im Ruhezustand der Schaltungsanordnung der Betrieb des Spannungsvervielfachers deaktiviert ist.

8. Schweißhelm oder Schweißmaske umfassend ein selbstverdunkelndes LCD-Element und die Schaltungsanordnung zur Ansteuerung des selbstverdunkelnden LCD-Elements nach einem der vorhergehenden Ansprüche.

9. Verfahren zur Ansteuerung eines selbstverdunkelndes LCD-Elements, wobei ein ruhiger Lichtbogen eine Situation bezeichnet, in der die Intensität des Lichts, das von einem beim TIG-Schweißen, Plasmalichtbogenschweißen oder Plasmaschneiden verwendeten Lichtbogen emittiert wird, besonders stet ist, wobei das Verfahren umfasst:
- Detektieren der Intensität von einfallendem Licht in Abhängigkeit von der Zeit und Erzeugen eines Detektionssignals (160) basierend auf der detektierten Intensität des einfallenden Lichts;
**gekennzeichnet dadurch, dass** das Verfahren umfasst:
- Verarbeiten des Detektionssignals (160) zur Erzeugung eines Ruhig-Lichtbogen-Anzeigesignals (162), das das Vorliegen eines ruhigen Lichtbogens anzeigt, wenn die Amplitudenschwankung des Detektionssignals (160) in einem für einen ruhigen Lichtbogen charakteristischen vorherbestimmten Frequenzbereich einen vorherbestimmten Schwellwert übersteigt, und wobei eine Untergrenze des vorherbestimmten Frequenzbereichs zwischen 300 Hz und 400 Hz beträgt, und
- Aktivieren der Verdunkelung des selbstverdunkelnden LCD-Elements, wenn vom Ruhig-Lichtbogen-Anzeigesignal (162) das Vorliegen des ruhigen Lichtbogens angezeigt wird.

10. Verfahren nach Anspruch 9, wobei das Verarbeiten des Detektionssignals (160) umfasst:
- Hochpassfiltern des Detektionssignals zur Extraktion eines Teils des Detektionssignals, der in einem für einen ruhigen Lichtbogen charakteristischen vorherbestimmten Frequenzbereich liegt, und wobei der vorherbestimmte Frequenzbereich oberhalb eines entsprechenden Frequenzbereichs der Intensitätsschwankung eines von typischen Umgebungslichtquellen, wie von Glühlampen, fluoreszierenden Lampen und/oder LED-Lampen emittierten Lichts liegt, und
- Vergleichen der Amplitude des gefilterten Detektionssignals (161) mit dem vorherbestimmten Schwellwert zur Erzeugung des Ruhig-Lichtbogen-Anzeigesignals (162).

11. Verfahren nach Anspruch 9 oder 10, ferner umfassend: Tiefpassfiltern des Detektionssignals, wobei die Tiefpassfilterung die Obergrenze des vorherbestimmten Frequenzbereichs definiert, wobei die Obergrenze zwischen etwa 1 kHz und etwa 3 kHz beträgt.

12. Verfahren nach einem der Ansprüche 9 bis 12, ferner umfassend: Aktivieren der Verdunkelung des selbstverdunkelnden LCD-Elements auch bei Feststellung des Vorliegens eines einfallenden Lichts mit einer eine vorherbestimmte Intensitätsschwelle überschreitenden Intensität.

13. Verfahren nach einem der Ansprüche 9 bis 13, ferner umfassend: Setzen einer das Verfahren implementierenden Schaltungsanordnung in einen der folgenden Zustände:
a) einen Aktiv-Zustand, in dem die Schaltungsanordnung aktiv ist und das selbstverdunkelnde LCD-Element abgedunkelt wird,
b) einen Standby-Zustand, in dem die Schaltungsanordnung aktiv und in der Lage ist, die Verdunkelung des selbstverdunkelnden LCD-Elements binnen etwa 0,1 bis 1 ms nach Empfang der Anzeige über das Vorliegen des ruhigen Lichtbogens und, in Abhängigkeit von Anspruch 12, bei Feststellung des Vorliegens von einfallendem Licht mit einer die vorherbestimmte Intensitätsschwelle übersteigenden Intensität, zu aktivieren, und
c) einen Ruhezustand, in dem die Schaltungsanordnung mindestens zum Teil in einen stromsparenden Zustand versetzt wird, in dem sie weniger Strom als im Aktiv-Zustand verbraucht.

14. Verfahren nach Anspruch 13, wobei die Schaltungsanordnung vom Ruhezustand in den Standby-Zustand versetzt wird, wenn festgestellt wird, dass der Bewegungsbetrag innerhalb einer vorherbestimmten Zeitperiode einen vorherbestimmten Bewegungsschwellwert überschreitet, und die Schaltungsanordnung vom Standby-Zustand in den Ruhezustand versetzt wird, wenn detektiert wird, dass der Bewegungsbetrag der Vorrichtung innerhalb der vorherbestimmten Zeitperiode den vorherbestimmten Bewegungsschwellwert nicht überschreitet.

15. Verfahren nach einem der Ansprüche 13 bis 14, wobei das Verfahren ferner das Erzeugen einer Triggerspannung zur Auslösung der Aktivierung des selbstverdunkelnden LCD-Elements umfasst, wobei
a) im Standby-Zustand die Triggerspannung periodisch erzeugt wird und die Triggerspannung während Ausschaltperioden des periodischen Triggerspannungserzeugungsbetriebs in mindestens einem Kondensator temporär gespeichert wird, und
b) im Ruhezustand der Schaltungsanordnung keine Triggerspannung erzeugt wird.

## Revendications

1. Dispositif comprenant un élément LCD auto-noircissant et un circuit pour piloter l'élément LCD auto-noircissant, dans lequel un arc calme désigne une situation dans laquelle l'intensité d'une lumière émise par un arc utilisé pour le soudage TIG, le soudage à l'arc au plasma ou le coupage au jet de plasma est particulièrement stable, ledit circuit comprenant :
- un photodétecteur (60) configuré pour détecter l'intensité de la lumière incidente en fonction du temps et pour générer un signal de détection (160) sur la base de l'intensité détectée de la lumière incidente,
**caractérisé en ce que** le circuit comprend :
- un montage de traitement de signal (61, 62) configuré pour traiter le signal de détection (160) afin de générer un signal indicateur d'arc calme (162) indiquant la présence d'un arc calme lorsque la variation de l'amplitude du signal de détection (160) dans une plage de fréquence prédéterminée caractéristique pour l'arc calme est supérieure à une valeur seuil prédéterminée, et dans lequel une limite inférieure de la plage de fréquence prédéterminée est comprise entre 300 Hz et 400 Hz, et
- un montage d'activation configuré pour activer le noircissement de l'élément LCD auto-noircissant lorsque la présence de l'arc calme est indiquée par le signal indicateur d'arc calme (162).

2. Dispositif selon la revendication 1, dans lequel ledit montage de traitement de signal (61, 62) comprend :
- un filtre passe-haut (61) configuré pour filtrer le signal de détection (160), une fréquence de coupure dudit filtre passe-haut (61) étant située à une limite inférieure de ladite plage de fréquence prédéterminée qui est caractéristique pour un arc calme, et ladite fréquence de coupure dudit filtre passe-haut (61) étant supérieure à une plage de fréquence respective de la variation d'intensité d'une lumière émise par des sources lumineuses ambiantes typiques, telles que lampes à incandescence, lampes à fluorescence et/ou lampes LED, et
- un comparateur (62) configuré pour comparer l'amplitude du signal de détection filtré (161) à la valeur seuil prédéterminée pour générer le signal indicateur d'arc calme.

3. Dispositif selon la revendication 1 ou 2, dans lequel le montage de traitement de signal comprend également un filtre passe-bas configuré pour filtrer en passe-bas le signal de détection, ledit filtre passe-bas ayant une fréquence de coupure comprise entre environ 1 kHz et environ 3 kHz, ou un filtre passe-bande configuré pour effectuer tant ledit filtrage en passe-haut que ledit filtrage en passe-bas.

4. Dispositif selon l'une des revendications précédentes, dans lequel le montage d'activation est également configuré pour activer le noircissement de l'élément LCD auto-noircissant lors de la détermination, par le circuit pour piloter l'élément LCD auto-noircissant, de la présence d'une lumière incidente avec une intensité qui dépasse un seuil d'intensité prédéterminé.

5. Dispositif selon l'une des revendications précédentes, dans lequel le circuit est configuré pour avoir
a) un état actif dans lequel le circuit est actif et l'élément LCD auto-noircissant est noirci,
b) un état d'attente dans lequel le circuit est actif et capable d'activer ledit noircissement de l'élément LCD auto-noircissant dans un délai d'environ 0,1 à 1 ms à partir de la réception de l'indication de présence de l'arc calme et, quand il dépend de la revendication 4, lors de la détermination de la présence d'une lumière incidente avec une intensité qui dépasse le seuil d'intensité prédéterminé, et
c) un état de repos dans lequel le circuit est, au moins en partie, mis dans un état de faible consommation d'énergie dans lequel il consomme moins d'électricité que dans l'état actif.

6. Dispositif selon la revendication 5, dans lequel le circuit comprend également un détecteur de mouvement, ledit circuit étant configuré pour être mis de l'état de repos à l'état d'attente lors de la détection, par le détecteur de mouvement, que la quantité de mouvement du dispositif au cours d'une période de temps prédéterminée dépasse une valeur seuil de mouvement prédéterminée, et ledit circuit étant configuré pour être mis de l'état d'attente à l'état de repos lors de la détection, par le seuil de mouvement, que la quantité de mouvement du dispositif au cours de la période de temps prédéterminée ne dépasse pas la valeur seuil de mouvement prédéterminée.

7. Dispositif selon l'une des revendications 5 à 6, dans lequel le circuit comprend également un multiplicateur de tension configuré pour générer une tension de déclenchement pour déclencher l'activation de l'élément LCD auto-noircissant, dans lequel
a) dans l'état d'attente du circuit, le multiplicateur de tension est configuré pour fonctionner de manière périodique pour générer la tension de déclenchement, et le circuit comprend également au moins un condensateur configuré pour emmagasiner temporairement la tension de déclenchement lorsque le multiplicateur de tension est à l'arrêt pendant le fonctionnement périodique de la génération de tension de déclenchement, et
b) dans l'état de repos du circuit, le fonctionnement du multiplicateur de tension est désactivé.

8. Masque de soudage ou casque de soudage comprenant un élément LCD auto-noircissant et le circuit pour piloter l'élément LCD auto-noircissant selon l'une des revendications précédentes.

9. Procédé pour piloter un élément LCD auto-noircissant, dans lequel un arc calme désigne une situation dans laquelle l'intensité d'une lumière émise par un arc utilisé pour le soudage TIG, le soudage à l'arc au plasma ou le coupage au jet de plasma est particulièrement stable, ledit procédé comprenant l'étape consistant à :
- détecter l'intensité d'une lumière incidente en fonction du temps et générer un signal de détection (160) sur la base de l'intensité détectée de la lumière incidente;
**caractérisé en ce que** le procédé comprend les étapes consistant à :
- traiter ledit signal de détection (160) pour générer un signal indicateur d'arc calme (162) indiquant la présence d'un arc calme lorsque la variation de l'amplitude du signal de détection (160) dans une plage de fréquence prédéterminée caractéristique pour un arc calme est supérieure à une valeur seuil prédéterminée, et une limite inférieure de ladite plage de fréquence prédéterminée étant comprise entre 300 Hz et 400 Hz, et
- activer le noircissement de l'élément LCD auto-noircissant lorsque la présence de l'arc calme est indiquée par le signal indicateur d'arc calme (162).

10. Procédé selon la revendication 9, dans lequel le traitement dudit signal de détection (160) comprend les étapes consistant à :
- filtrer en passe-haut le signal de détection pour extraire une portion du signal de détection qui est située dans une plage de fréquence prédéterminée qui est caractéristique pour un arc calme, et ladite plage de fréquence prédéterminée étant supérieure à une plage de fréquence respective de la variation d'intensité d'une lumière émise par des sources lumineuses ambiantes typiques, telles que lampes à incandescence, lampes à fluorescence et/ou lampes LED, et
- comparer l'amplitude du signal de détection filtré (161) à la valeur seuil prédéterminée pour générer le signal indicateur d'arc calme (162).

11. Procédé selon la revendication 9 ou 10, comprenant également l'étape consistant à filtrer en passe-bas le signal de détection, ledit filtrage passe-bas définissant la limite supérieure de la plage de fréquence prédéterminée, ladite limite supérieure étant comprise entre environ 1 kHz et environ 3 kHz.

12. Procédé selon l'une des revendications 9 à 12, comprenant également l'étape consistant à activer ledit noircissement de l'élément LCD auto-noircissant également lors de la détermination de la présence d'une lumière incidente avec une intensité qui dépasse un seuil d'intensité prédéterm iné.

13. Procédé selon l'une des revendications 9 à 13, comprenant également l'étape consistant à mettre un circuit mettant en oeuvre le procédé dans l'un parmi :
a) un état actif dans lequel le circuit est actif et l'élément LCD auto-noircissant est noirci,
b) un état d'attente dans lequel le circuit est actif et capable d'activer ledit noircissement de l'élément LCD auto-noircissant dans un délai d'environ 0,1 à 1 ms à partir de la réception de l'indication de la présence de l'arc calme et, quand il dépend de la revendication 12, lors de la détermination de la présence d'une lumière incidente avec une intensité qui dépasse le seuil d'intensité prédéterminé, et
c) un état de repos dans lequel le circuit est, au moins en partie, mis dans un état de faible consommation d'énergie dans lequel il consomme moins d'électricité que dans l'état actif.

14. Procédé selon la revendication 13, dans lequel le circuit est mis de l'état de repos à l'état d'attente lors de la détermination que la quantité de mouvement au cours d'une période de temps prédéterminée dépasse une valeur seuil de mouvement prédéterminée, et le circuit est mis de l'état d'attente à l'état de repos lors de la détection que la quantité de mouvement du dispositif au cours de la période de temps prédéterminée ne dépasse pas la valeur seuil de mouvement prédéterminée.

15. Procédé selon l'une des revendications 13 à 14, ledit procédé comprenant également l'étape consistant à générer une tension de déclenchement pour déclencher l'activation de l'élément LCD auto-noircissant, dans lequel
a) dans l'état d'attente, la tension de déclenchement est générée périodiquement, et la tension de déclenchement est emmagasinée temporairement dans au moins un condensateur pendant des périodes d'arrêt du fonctionnement périodique de génération de la tension de déclenchement, et
b) dans l'état de repos du circuit, la tension de déclenchement n'est pas générée.
